# NEUE EUROPÄISCHE PATENTSCHRIFT

(11) **EP 1 421 894 B2**
(45) Veröffentlichungstag und Bekanntmachung des Hinweises auf die Entscheidung über den Einspruch: **23.02.2011**
(45) Hinweis auf die Patenterteilung: 23.01.2008
(21) Anmeldenummer: 03026488.1
(22) Anmeldetag: 20.11.2003
(51) Int. Cl.: A61B 1/00, A61B 1/05

(54) **Endoskopkopf**
Endoscope head
Tête d'endoscope

(30) Priorität: 22.11.2002 DE 10254609
(43) Veröffentlichungstag der Anmeldung: 26.05.2004
(73) Patentinhaber: Invendo Medical GmbH, 69469 Weinheim (DE)
(72) Erfinder: Viebach, Thomas, 82282 Pischertshofen (DE); Pauker, Fritz, 86316 Friedberg (DE)
(74) Vertreter: Winter, Brandl, Fürniss, Hübner, Röss, Kaiser, Polte - Partnerschaft

(56) Entgegenhaltungen:
- EP-A- 0 531 777
- EP-A- 1 104 182
- EP-A- 1 371 321
- WO-A-02/056756
- DE-A1- 19 806 984
- DE-A1- 19 827 255
- DE-A1- 19 839 188
- US-A- 5 371 384
- US-A- 5 398 670
- US-A- 5 643 175
- US-B1- 6 447 445

## Beschreibung

Die Erfindung bezieht sich auf einen Endoskopkopf mit mehreren Funktionseinheiten oder Elementen gemäß dem Oberbegriff des Patentanspruchs 1.

Aus US 6 447 445 ist ein Endoskop gemäß dem Stand der Technik bekannt.

Endoskope kommen insbesondere in der Medizin zur diagnostischen Betrachtung (Spiegelung) von Körperhöhlen und Hohlorganen zum Einsatz. Dabei sind aus dem Stand der Technik flexible Endoskope hinlänglich bekannt, die einen Arbeitsgang zum Einführen von Arbeitsgeräten und einen Endoskopkopf haben, der mit Beleuchtungseinrichtungen, Bildübertragungseinrichtungen und anderen Einrichtungen ausgestattet sein kann.

Es ist jedoch auch seit Einführung dieser Endoskope bekannt, dass auf Grund deren unsachgemäßer Reinigung, Desinfektion und Sterilisation, bei mehrfacher Verwendung der Endoskope, beispielsweise Microorganismen übertragen werden können die ihrerseits wiederum Krankheiten von einem Patienten auf den anderen Patienten übertragen können. Eine ausreichende Desinfektion und Sterilisation des Endoskops stellt einen erheblichen Arbeits- und Kostenaufwand dar, bzw. scheint es auch Erreger zu geben die gegenüber den herkömmlichen Desinfektionsmethoden besonders resistent sind.

Prinzipiell werden daher Endoskope mit Endoskopköpfen versehen, bestehend aus einer Anzahl von elektrischen, optischen und hydraulischen Funktionselementen, die auf einer Halterung platziert und anschließend mit einem Körperverträglichen Material wie beispielsweise Silikon umgossen werden. Dieser Vorgang wird derart ausgeführt, dass auch gleichzeitig das distale Ende des Endoskopschafts, auf welchem der Kopf montiert ist, mit umgossen wird, um den Übergang zwischen dem Endoskopkopf und dem Endoskopschaft abzudichten.

Es hat sich gezeigt, dass die herkömmliche Herstellungsweise eines solchen Endoskops insbesondere bedingt durch den Aufbau des Endoskopkopfs sehr teuer ist und daher und mehrfach verwendbare Endoskope wirtschaftlich sind. Diese haben jedoch wiederum den Nachteil, ständig aufwendig desinfiziert werden zu müssen.

Um die vorstehenden Schwierigkeiten, Risiken und damit verbundenen Kostenaufwendungen zu umgehen, ist es eine Aufgabe der Erfindung, ein Einwegendoskop, insbesondere einen Kopf für ein Einwegendoskop zu schaffen, der so einfach und kostengünstig hergestellt werden kann, dass er nach Gebrauch entsorgt werden kann.

Diese Aufgabe wird mit den Merkmalen des Anspruchs 1 gelöst. Der Kern der Erfindung besteht demzufolge darin, dass der Endoskopkopf aus einer Anzahl von modularen Funktionsträgern für das Aufnehmen und/oder Ausbilden entsprechend zugeordneter Funktionseinheiten aufgebaut ist, die das Endoskop entsprechend seinem vorgesehenen Einsatzzweck aufweisen muss. Die Funktionsträger werden vor der Montage mit den entsprechenden Funtionseinheiten oder -elementen bestückt und anschließend vorzugsweise durch Schnapp- oder Klemmverbindungen zusammengesetzt.

Für den Anbau des Kopfes an ein Endoskopschaft eine Art Zwischenstück oder Montageadapter vorgesehen, der eine Verbindung zwischen den Funktionselementen des Kopfes und den entsprechenden Leitungen und Kanälen im Endoskopschaft herstellt bzw. ermöglicht.

Schließlich ist die Schutzkappe zumindest teilweise aus einem lichtdurchlässigen Material aufgebaut, wobei die elektronischen Bauteile auch einen opt. Sensorchip Leuchtkörper umfassen.

Durch den modularen Aufbau des Kopfes können die modularen Einzelteile sehr kostengünstig hergestellt und der Kopf montiert werden. Durch die Reduktion der Fertigungskosten des Kopfes kann somit ein Endoskop geschaffen werden, das als Einwegendoskop verwendet werden kann.

Weitere vorteilhafte Ausgestaltungen der Erfindung sind Gegenstand der übrigen Patentansprüche.

Die Erfindung wird nachstehend anhand bevorzugter Ausführungsbeispiele unter Bezugnahme auf die beiliegenden schematischen Figuren näher beschrieben.
Fig. 1 zeigt eine isometrische Teilschnittansicht eines erfindungsgemäßen Endoskopkopfs von schräg oben;
Fig. 2a bzw. 2b zeigt eine Unterseite bzw. Oberseite eines dem Endoskopkopf gemäß Fig. 1 zugehörigen Trägerelements für elektrische Bauteile;
Fig. 3 zeigt eine Perspektivenansicht der Oberseite eines erfindungsgemäßen Montageadapters;
Fig. 4 zeigt eine Perspektivenansicht der Unterseite des erfindungsgemäßen Montageadapters von Fig. 3 und
Fig. 5 zeigt eine Perspektivenansicht eines weiteren Ausführungsbeispiels eines erfindungsgemäßen Endoskopkopfes.

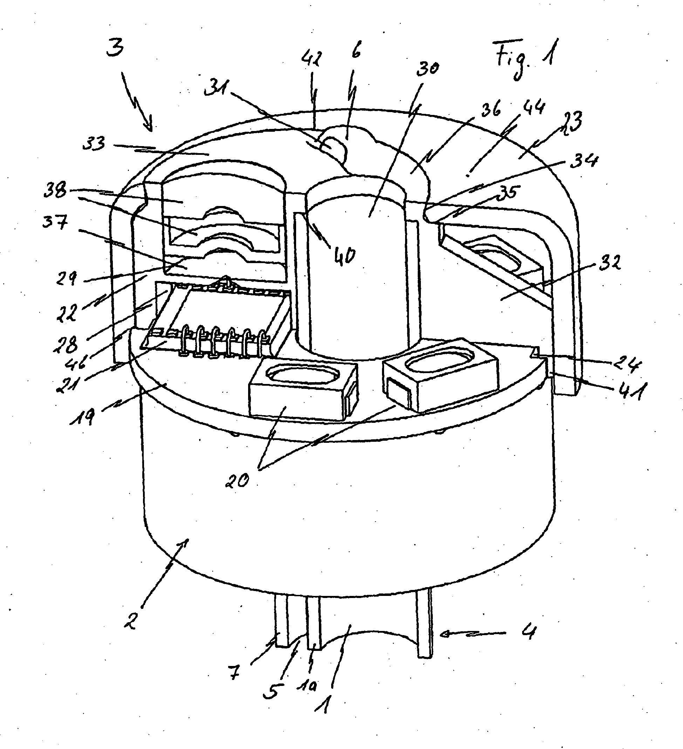

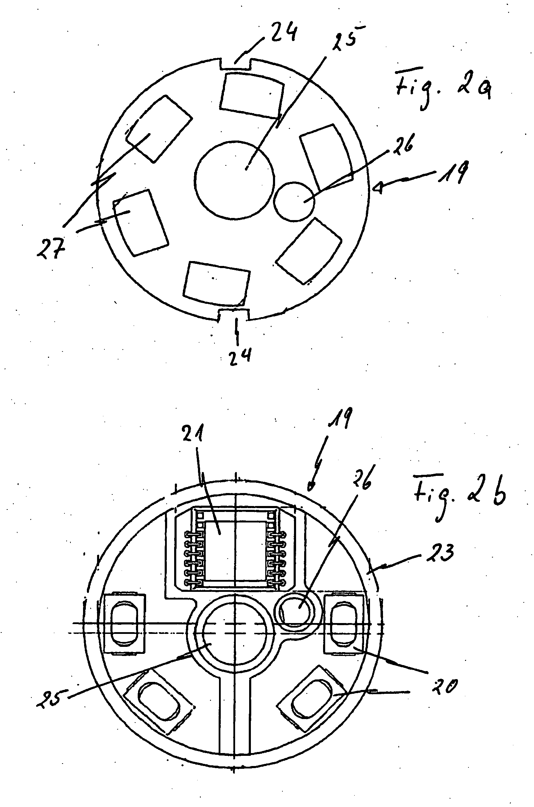

Anhand von Figuren 1 bis 4 wird ein Endoskop mit Endoskopkopf gemäss einem Ausführungsbeispiel der Erfindung beschrieben.

Das Endoskop besteht im Wesentlichen aus einem (nicht dargestellten) flexiblen Endoskopschaft oder -schlauch, der einen zentral verlaufenden Arbeitskanal 1 aufnimmt, einem an dem einen distalen Ende des Endoskopschlauchs befestigten Montageadapter 2 und einem Endoskopkopf 3, der über den Montageadapter 2 an dem Endoskopschlauch befestigt ist. Der erfindungsgemäße Endoskopkopf 3 wiederum besteht im Wesentlichen aus einer Anzahl von modularen Funktionsträgern 19, 22, 23 für das Aufnehmen und/oder Ausbilden entsprechend zugeordneter bestimmter Funktionseinheiten 20, 21, 38, die der Endoskopkopf für die Durchführung beispielsweise einer Untersuchung an einem menschlichen Körperhohlraum benötigt.

Als derartige Funktionsträger sind in diesem bevorzugten Ausführungsbeispiel vorgesehen:
- Ein Trägerelement 19 zur Aufnahme von elektronischen Bauteilen 20, 21,
- ein eine Optik aufnehmendes/ausbildendes Halterungselement 22 und
- eine Schutzkappe 23 zur Abdeckung der beiden vorstehende Funktionsträger.

Der Endoskopschlauch ist für die hierin beschrieben Erfindung insofern interessant, als dass der Montageadapter 2 quasi als Zwischenstück für die Aufnahme bzw. Verknüpfung bestimmter Elemente des Endoskopschlauchs mit dem Endoskopkopf ausgebildet ist. So verfügt der Endoskopschlauch über (nicht dargestellte) Abkrümmungselemente zum Abkrümmen seines distalen Endbereichs, über (ebenfalls nicht dargestellte) strom- oder lichtleitende Kabel oder Leitungen, die die elektronischen Bauteile 20, 21 im Endoskopkopf 3 mit Strom versorgen, bzw. durch die Informationen übertragen werden können und über einen Doppelkanal 4 bestehend aus einem innenliegenden Arbeitskanal 1 zum Einführen von Arbeitsinstrumenten in den zu betrachtenden Hohlraum und aus einem außenliegenden Spülkanal 5 zum Versorgen einer an dem Endoskopkopf 3 angebrachten Spüldüse 6 mit Spülflüssigkeit, welche eine weitere Funktionseinheit darstellt. Dabei ist diese Kanalanordnung als aus zwei zylindrischen Kanalelementen oder - rohren 1a, 7 mit unterschiedlichen Außendurchmessern bestehend zu verstehen, wobei das kleinere Kanalelement 1a so in dem größeren Kanalelement 7 liegt, dass sich deren Kanalwände an einer Längsseite tangieren, d.h. sie sind coaxial, jedoch achsversetzt zueinander angeordnet. Somit entsteht aus dem inneren Kanalelement 1a der Arbeitskanal 1 und aus dem Bereich zwischen dem äußerem 7 und innerem Kanalelement 1a entsteht der, in diesem Fall sichelförmige Spülkanal 5. Auf Grund dieser Anordnung haben die beiden Kanalelemente 1a, 7 unterschiedliche Längsachsen, die um die Differenz ihrer Radien (die im weiteren Verlauf als der Achsversatz bezeichnet wird) versetzt sind. Bezüglich der Länge ist das innere Kanalelement 1a um einen durch die Höhe des Endoskopkopfs bestimmten Betrag länger als das äußere Kanalelement 7. Somit wird der Doppelkanal 4 ab einer bestimmten Stelle lediglich von dem Arbeitskanal 1 weitergeführt.

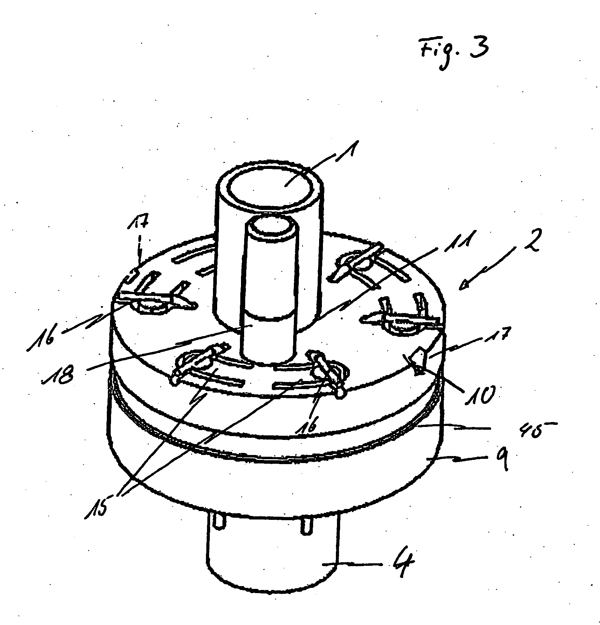

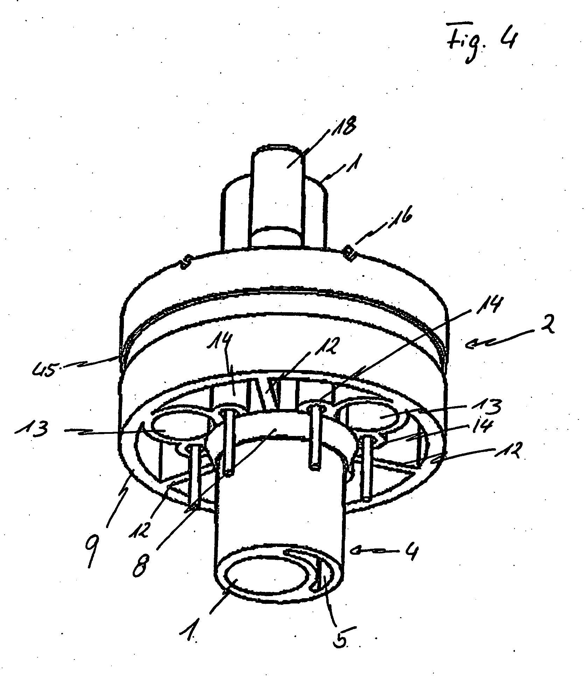

Der Montageadapter 2 gemäß der Fig. 3 und 4 besteht im Wesentlichen aus zwei konzentrisch ineinanderliegenden Zylinderelementen 8, 9 mit unterschiedlichen Radien, die über eine zu der Zylinderachse senkrecht stehende Adapterplatte 10 miteinander verbunden sind, die auf einer Stirnseite beider Zylinderelemente 8, 9 vorzugsweise einstückig mit diesen ausgebildet ist. Dabei hat das äußere Zylinderelement 9 einen Außendurchmesser der im Wesentlichen dem Außendurchmesser des Endoskopschlauchs entspricht und der Innendurchmesser des inneren Zylinderelements 8 entspricht im wesentlichen dem Außendurchmesser des Doppelkanals 4. Ein Kreisloch 11 in der Adapterplatte 10 hat jedoch einen Innendurchmesser der dem Außendurchmesser des Arbeitskanals 1 entspricht und sein Mittelpunkt ist mit Bezug auf den Mittelpunkt der Zylinderelemente 8, 9 um den Versatz der Kanalelemente versetzt. Somit entsteht im Übergang zwischen dem inneren Zylinderelement 8 und der Adapterplatte 10 ein sichelförmiger Überstand, der als Auflager des Montageadapters 2 auf den Doppelkanal 4 dient.

Der kreissegmentartige Hohlraum zwischen den Zylinderelementen 8, 9 ist durch radiale Rippen 12 in drei Abschnitte geteilt und in der Mitte jedes Abschnitts befindet sich ein zylindrisches Aufnahmemittel 13 zum Aufnehmen der Abkrümmungselemente, welches sich axial erstreckt und dessen Wandung sowohl mit dem inneren 8 als auch mit dem äußeren 9 konzentrischen Zylinderelement des Adapters 2 in Kontakt ist. Beidseitig der Aufnahmemittel 13 sind jeweils zwei zylindrische Kabelführungen 14 angeordnet, die sich ebenfalls axial erstrecken und deren Wandung sowohl mit dem inneren Zylinderelement 8 als auch mit dem Aufnahmemittel 13 in Kontakt ist.

Im Bereich, in dem die Kabelführungen 14 auf die Adapterplatte 10 stoßen, sind in der Adapterplatte 10 in Axialrichtung federnde, sich tangential erstreckende Kontaktarme 15 innerhalb der Dicke der Adapterplatte 10 ausgebildet, an deren Unterseiten durch die Kabelführungen 14 hindurch führende Kabel anschließbar sind und deren Oberseiten mit Kontaktstellen (Pads) 16 versehen sind, die über die Oberfläche der Adapterplatte 10 überstehen.

Außerdem sind an der Aussenkante der Montageplatte 10 zwei gegenüberliegende Einkerbungen 17 vorgesehen, die sowohl die Adapterplatte 10 an ihrem Rand als auch das äußere Zylinderelement 9 einkerben und die sich radial gegenüberliegen. Überdies ist an dem Außenrand des äußeren Zylinders 9 eine ringförmige Vertiefung 45 vorgesehen.

Des weiteren verfügt der Montageadapter 2 an seiner Montageplatte 10 über ein Spülflüssigkeitsrohr 18, das sich parallel zu dem und im Bereich des Arbeitskanals 1 ohne Spülkanal erstreckt, wenn der Montageadapter 2 auf den Endoskopschlauch gesteckt ist und das über eine Öffnung in der Adapterplatte 10 mit dem Spülkanal 5 verbunden ist.

Durch das vorstehend beschriebene Verhältnis zwischen dem Innendurchmesser des inneren Zylinderelements 8 und dem Innendurchmesser des Adapterplattenkreislochs 11 zu den jeweiligen Durchmessern des Arbeitskanals 1 bzw. Doppelkanals 4, kann der Montageadapter 2 nun einfach auf den Arbeitskanal 1 geschoben werden, wobei der an dem Übergang zwischen der Adapterplatte 10 und dem inneren Zylinderelement 8 sichelförmige Überstand auf einem Übergang zwischen Arbeitskanal mit Spülkanal (Doppelkanal) 4 und ohne Spülkanal 1 aufliegt, die Kabel durch die Kabelführungen 14 mit den Kontaktstellen 16 und die Abkrümmungselemente des Endoskopschlauchs mit den Aufnahmemitteln 13 verbunden werden können.

An dieser Stelle sei darauf hingewiesen, dass in der vorstehenden Beschreibung teilweise Bezug auf den Endoskopkopf gemäß diesem Ausführungsbeispiel genommen wurde, wobei jedoch der erfindungsgemäße Montageadapter auch für andere, bereits existierende Endoskopköpfe für deren Montage an einem Endoskopschaft verwendet werden kann.

Im folgenden wird nunmehr der erfindungsgemäße Endoskopkopf insbesondere Anhand der Fig. 3 und 4 detaillierter beschrieben.

Das Trägerelement 19 hat eine Plattenform die im Wesentlichen der Form der Montageplatte 10 entspricht, die Achse der Aussenkante und die Achse des inneren Kreislochs 25 der Trägerplatte 19 sind also wieder um den Versatz der beiden Kanalelemente 1, 7 zueinander versetzt. Außerdem hat es an seinem äußeren Rand zwei Einkerbungen 24 und ein exzentrisches Spülrohrloch 26 ist durch das Trägerelement 19 hindurch gearbeitet, durch das das Spülrohr 18 hindurch geführt werden kann. Wenn das Trägerelement 19 auf der Montageplatte 10 aufliegt und dabei das Spülrohr 18 durch das exzentrische Spülrohrloch 26 des Trägerelements 19 durchgeführt ist, stehen die Einkerbungen 24 in der Trägerplatte und die Einkerbungen 17 in der Montageplatte, sowie das Kreisloch 11 der Montageplatte und das Kreisloch 25 der Trägerplatte jeweils bündig zueinander.

An der Unterseite des Trägerelements 19 sind in gleichmäßigem Abstand zueinander sechs Kontaktfelder 27 angeordnet, die mit den Kontaktstellen 16 der Montageplatte 10 in Kontakt sind, wenn das Trägerelement 19 auf der Montageplatte 10 aufliegt und die Einkerbung 17, 24 jeweils bündig zueinander stehen. Die Kontaktfelder 27 stellen einen elektrischen Kontakt zwischen der Unterseite und der Oberseite der Trägerplatte 19 her. An der Oberseite der Trägerplatte befinden sich vier Leuchtkörper 20 die jeweils mit einem der Kontaktfelder 27 in Verbindung sind und ein optischer Sensorchip 21 ist mit einem weiteren Kontaktfeld 27 des Trägerelements in Kontakt, das sich in der Nähe einer der beiden Einkerbungen 24 befindet. Zieht man eine Linie zwischen den Mitten der gegenüberliegen Einkerbungen 24, so halbiert die Linie sowohl die Trägerplatte 19 als auch den optischen Sensorchip 21 und jeweils zwei Leuchtkörper 20 sind symmetrisch zu dieser Linie angeordnet.

Das Halterungselement für die Optik 22 besteht im Wesentlichen aus einer kubischen Sensorchipkammer 28, einer darüber liegenden, durch eine Trennwand von der Sensor- oder Kamerachipkammer 28 abgeteilten, zylindrischen Linsenkammer 29, einer sich neben den beiden Kammern in Axialrichtung erstreckenden zylindrischen Arbeitskanaldurchführung 30 und einer sich parallel dazu erstreckenden zylindrischen Spülrohrdurchführung 31. Des weiteren ist seitlich der Arbeitskanaldurchführung 30 eine abgeschrägte Versteifungsrippe 32 angeordnet. Die übereinanderliegenden Kammern 28, 29, die Arbeitskanaldurchführung 30 und die Rippe 32 sind jeweils bezüglich einer gemeinsamen zu dem Trägerelement 19 senkrechtstehenden und das Trägerelement 19 halbierenden Ebene symmetrisch. Die Spülrohrdurchführung 31 ist außerhalb dieser Ebene angeordnet. Wie ferner aus der Fig. 3 zu entnehmen ist, sie das Halterungselement 22 einstückig ausgebildet.

Bis zu einer gewissen Höhe hat das Halterungselement 22 eine den Formen der zugehörigen Kammern 28, 29 und Durchführungen 30, 31 entsprechende, diese Formen einhüllende Außenkontur. Ab der gewissen Höhe ändert sich die Außenkontur in die Form einer runden Scheibe 33, die an ihrem oberen Ende einen Rücksprung 34 hat. Somit entstehen hier zwei übereinanderliegende, konzentrische, runde Scheibenabschnitte 35, 36 wobei der obere Scheibenabschnitt 36 nach oben leicht konisch auseinander geht. Ein Schnitt durch diesen Rücksprung 34 ergibt im wesentlichen eine L-Form, deren stehender Schenkel geringfügig geneigt ist, sodass ein leicht spitzer Winkel zwischen den Schenkeln entsteht.

Die Höhe des Rücksprungs 34 entspricht im Wesentlichen der Dikke einer später beschriebenen Schutzkappe 23.

Die Sensorchipkammer 28 ist nach unten offen und hat solche Abmessungen, dass sie den Sensorchip 21 aufnehmen bzw. diesen umgeben kann. Darüber liegend ist die zylindrische Linsenkammer 29 angeordnet und beide Kammern sind durch eine Wand 37 mit einem mittigen Loch voneinander getrennt. Die Linsenkammer 29 nimmt zumindest eine optische Linse 38 bzw. ein Linsensystem auf, die/das wahlweise zoombar ausgeführt sein kann und entweder unmittelbar an Linsenhalterungen fixiert sind oder in Form einer vorgefertigten Patrone in die Kammer in die Kammer einführbar ist. Die Linsenkammer 29 ist nach oben offen, sie ist jedoch vorzugsweise mit einer lichtdurchlässigen Abdeckung 39 abgedeckt.

Die Arbeitskanaldurchführung 30 hat bis zu einer bestimmten Höhe einen Innendurchmesser, der dem Außendurchmesser des Arbeitskanals 1 ohne Spülkanal entspricht. An seinem Ende befindet sich ein Rücksprung 40, dessen Rücksprungtiefe der Dicke des Arbeitskanals 1 im Bereich ohne Spülkanal entspricht.

Die Spülrohrdurchführung 31 hat einen Innendurchmesser, der im Wesentlichen dem Außendurchmesser des Spülrohrs 18 entspricht und endet am oberen Ende des Linsenhalterungselements 22 in der Düse 6, die auf die Abdeckung der Linsenkammer 29 gerichtet ist.

Außerdem hat das Halterungselement 22 für die Optik an der äußeren, unteren Seite der Rippe 32 und an der entgegengesetzten Seite dazu, an die Sensorchipkammer 28 angrenzend, jeweils einen Klammervorsprung 41, der mit jeweils einer Einkerbung der Trägerplatte 24 eingreifbar ist und somit eine feste, mechanische Verbindung dazwischen hergestellt werden kann. Der Abstand zwischen dem Mittelpunkt zwischen den Klammern 41 und der Achse der Arbeitskanaldurchführung 30 entspricht wiederum dem Versatz der beiden Kanalelemente 1, 7.

Die Schutzkappe 23 hat im Wesentlichen die Form einer umgedrehten Tasse oder Becher. Ihr Innendurchmesser entspricht dem Außendurchmesser des Trägerelements 19 und ihr "Tassenboden" 44 weist eine runde Öffnung 42 auf, deren Durchmesser dem Durchmesser der oberen, konischen Scheibe 36 des Linsenhalteelements 22 auf halber Höhe entspricht. Die Innenkante der Öffnung 42 im "Tassenboden" ist abgerundet und die Schutzkappe 23 besteht zumindest teilweise aus einem Lichtdurchlässigen Material und ist insgesamt fester als das Material des Halterungselements 22 für die Optik. Ferner hat die Schutzkappe 23 an ihrer Innenwandung einen ringförmigen Vorsprung 46 vorgesehen.

Nun wird der Zusammenbau des Endoskopkopfs 3 beschrieben.

Das Trägerelement 19 wird, wie dies vorstehend beschrieben ist, mit den Leuchtkörpern 20 und dem optischen Sensorchip 21 bestückt. Dann wird das, die optische Linse 38 haltende Halterungselement 22 auf die Trägerplatte 19 aufgesetzt, wobei die Klammern 41 des Linsenhalterungselements mit den Einkerbungen 24 in der Trägerplatte in Eingriff gelangen und eine mechanische Verbindung dazwischen hergestellt wird. Das Halterungselement 22 für die Optik sitzt nun so auf, dass der optische Sensorchip 21 durch die Sensorchipkammer 28 abgedeckt wird und Licht von Außen durch die Linsenkammerabdeckung 39, die Linse 38 und das Loch in der Trennwand 37 hindurch auf den optischen Sensorchip fallen kann. Gleichzeitig ist die Innenfläche der Arbeitskanalführung 30 bündig mit der Innenfläche des inneren Lochs 25 in dem Trägerelement 19 und die Innenfläche des Spülrohrdurchlasses 31 ist bündig mit der Innenfläche des exzentrischen Spülrohrlochs 26 in dem Trägerelement 19. Die Leuchtkörper 20 auf der Trägerplatte 19 werden von dem aufgesteckten Halterungselement 22 nicht beeinträchtigt.

Jetzt kann die Schutzkappe oder -hülle 23 an der zusammengebauten Anordnung von Trägerelement 19 und Linsenhalterungselement 22 befestigt werden. Die Anordnung wird mit dem Linsenhalterungselement 22 voraus in die tassenförmige Schutzkappe 23 eingebracht wobei der obere, konische Scheibenabschnitt 36 auf die Öffnung 42 im "Tassenboden" ausgerichtet wird und die Schutzhülleninnenwand auf den Umfang des Trägerelements 19 ausgerichtet wird. Auf Grund der konischen Ausbildung des oberen Scheibenabschnitts 36, der Abrundung der Kante der Öffnung im "Tassenboden" der Schutzhülle 23 und der Tatsache, dass das Linsenhalterungselement 22 aus einem weicheren Material als die Schutzhülle 23 gefertigt ist, kann die Schutzhülle 23 an das Linsenelement 22 gedrückt werden, wodurch zwischen dem konischen Scheibenabschnitt 36 und der Öffnung 42 der Schutzhülle 23 eine mechanische Verbindung entsteht, die die Schutzhülle 23 mit dem Linsenhalterungselement 22 und der daran befestigten Trägerplatte 19 zusammenhält. Somit ist ein zusammengebauter Endoskopkopf 3 entstanden, der nun über den Montageadapter 2 mit dem Endoskopschlauch verbunden werden kann, wie dies nachstehend beschrieben ist.

Wie schon erwähnt, ist der Montageadapter 2 mit dem Endoskopkopf 3 verbunden, wobei der Bereich des Arbeitskanals 1 ohne Spülkanal von dem Montageadapter 2 hervorsteht. Der zusammengebaute Endoskopkopf 3 kann nun auf den Montageadapter 2 aufgesetzt werden, indem der Arbeitskanal 1 ohne Spülkanal durch das Kreisloch 25 des Trägerelements in die Arbeitskanaldurchführung 30 eingeführt wird, wobei gleichzeitig das Spülrohr 18 des Adapters 2 durch das Spülrohrloch 26 der Trägerplatte 19 hindurch in die Spülrohrführung 31 eingeführt wird und die Klammern 41 des Linsenhalterungselements 22 in die Einkerbungen 17 des Montageelements 2 eingebracht werden. Die Länge des von dem Montageadapter 2 vorstehenden Arbeitskanals 1 ist so gewählt, dass sein Ende im Zusammenbauzustand an dem Rücksprung 40 der Arbeitskanaldurchführung 30 anliegt. Der Vorsprung 46 der auf den Montageadapter 2 aufgebrachten Schutzhülle 23 kommt mit dem Rücksprung 45 des Montageadapters 2 in Eingriff und stellt somit eine mechanische Verbindung zwischen dem Endoskopkopf 3 und dem Montageadapter 2 her.

In der auf diese Weise zusammengebauten Anordnung aus Endoskopkopf 3 und Montageadapter 2 stehen die Kontaktflächen 27 der Trägerplatte 19 nun in sicherem Kontakt mit den Kontaktstellen 16 des Montageadapters 2, da die Unterfläche der Trägerplatte 19 bündig auf der Oberfläche des Montageadapters 2 aufliegt und somit die federnden Kontaktstellenarme 15 leicht nach unten gedrückt werden, weil die Kontaktstellen 16 geringfügig über die Oberfläche des Montageadapters 2 hervorstehen. Die Kontaktstellen 16 werden im zusammengebauten Zustand somit sozusagen gegen die Kontaktflächen 27 vorgespannt.

Somit lässt sich ein Endoskop herstellen, wobei die den Endoskopkopf aufbauenden Funktionsträger, also die Trägerplatte 19, das Linsenhalterungselement 22 und die Schutzhülle 23, getrennt von einander gefertigt werden können, dann nach deren Bestükkung mit den ausgewählten Funktionseinheiten zu dem Endoskopkopf 3 zusammen gebaut werden können und der Endoskopkopf 3 schließlich einfach über den Montageadapter 2 auf dem Endoskopschlauch befestigt werden kann.

Im folgenden werden Abwandlungen der Erfindung beschrieben.

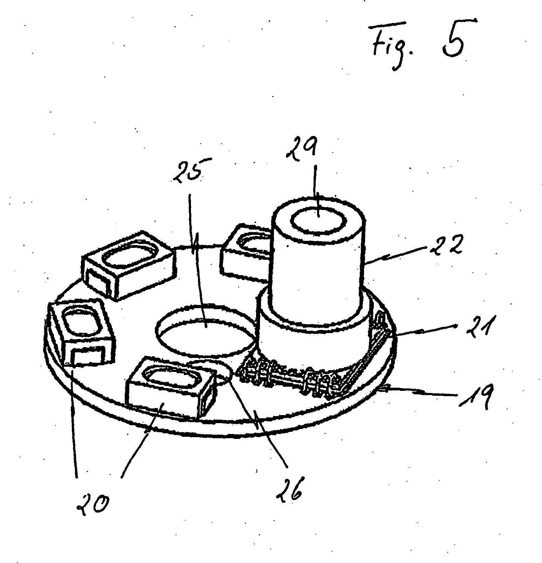

In einem anderen Ausführungsbeispiel der Erfindung gemäß der Fig. 5 kann das Linsenhalterungselement 22 auch lediglich als über dem optischen Sensor 21 angebrachtes, zylindrisches Element ausgebildet sein, ohne dabei den Arbeitskanal und das Spülrohr aufzunehmen. Vielmehr werden in diesem Ausführungsbeispiel der Arbeitskanal und das Spülrohr parallel zu dem Linsenhalterungselement liegend zu der Schutzhülle geführt und enden dort.

Schließlich sei noch darauf hingewiesen dass das Halterungselement zusätzlich mit einer nach Außen, d.h. über die Schutzkappe hinausragenden kippbaren und/oder drehbaren Spiegelungs- und/oder Prismenvorrichtung ausgerüstet sein kann, die bezüglich der die Optik aufnehmenden Kammer 29 so bewegbar ist, dass sie wahlweise Lichtstrahlen, die außerhalb des normalen Lichteinfallbereichs der in der Kammer befindlichen Optik liegen, auf die Optik umlenkt. Auf diese Weise läßt sich der Blickwinkel der Optik zur Seite oder gar rückwärts ähnlich eines verstellbaren Rückspiegels wahlweise erweitern.

Die Erfindung betrifft einen Endoskopkopf, der mit einer Anzahl von Funktionseinheiten wie beispielsweise Optik, Beleuchtungselemente, Spüldüsen und dergleichen ausgerüstet ist. Der Endoskopkopf besteht dabei im wesentlichen aus einer Anzahl von modularen, zusammensteck- oder klickbaren Funktionsträgern 19, 22, 23 für das Aufnehmen und/oder Ausbilden entsprechend zugeordneter Funktionseinheiten 20, 21, 38.

## Patentansprüche

1. Endoskopkopf, der mit einer Anzahl von Funktionseinheiten wie beispielsweise Optik, Beleuchtungselemente, Spüldüsen und dergleichen ausgerüstet ist, wobei der Endoskopkopf aus einer Anzahl von modularen Funktionsträgern (19, 22, 23) für das Aufnehmen und/oder Ausbilden entsprechend zugeordneter Funktionseinheiten (20, 21, 38) besteht, welche ein plattenförmiges Trägerelement (19) mit elektronischen Bauteilen (20, 21) nämlich einem optischen Sensor chip (21), ein Halterungselement (22) für eine zumindest zwei Linsen (38) aufweisende Optik; eine Schutzkappe (23) zum Abdecken des Linsenhalterungselements (22) und der Trägerplatte (19) und einen Montageadapter (2) zur Befestigung des Endoskopkopfs an einem Endoskopschaft ausbilden, wobei der Montageadapter (2) derart ausgebildet ist, um eine Verbindung zwischen im Endoskopschaft ausgebildeten Versorgungskanälen und Leitungen mit den Funktionseinheiten des Endoskopkopfs zu schaffen und/oder zu herzustellen, **dadurch gekennzeichnet, dass** die Schutzkappe (23) zumindest teilweise aus einem lichtdurchlässigen Material besteht und dass die elektronischen Bauteile (20, 21) ferner Leuchtkörper (20) umfassen.

2. Endoskopkopf nach Anspruch 1, **dadurch gekennzeichnet, dass** die Funktionsträger (19, 22, 23) derart ausgestaltet sind, dass die Funktionselemente (20, 21, 38) durch Zusammensetzen der Funktionsträger (19, 22, 23) vorzugsweise selbsttägig korrekt platzierbar und/oder in Funktionsfähigkeit bringbar sind.

3. Endoskopkopf gemäß Anspruch 2, **dadurch gekennzeichnet, dass** die Funktionsträger (19, 22, 3) mit jeweils in vorbestimmten Eingriff miteinander bringbaren, vorzugsweise elastisch deformierbaren Verbindungsabschnitten für eine Schnapp- und/oder Klemmverbindung ausgebildet sind.

4. Endoskopkopf nach Anspruch 1, **dadurch gekennzeichnet, dass** der Montageadapter (2) mit elektrischen Kontaktstellen (16) ausgebildet ist, und die elektrische Leitungen im Endoskopschaft anschließbar sind.

5. Endoskopkopf gemäß Anspruch 4, **dadurch gekennzeichnet, dass** das Trägerelement (19) an seiner zum Montageadapter (2) angewandten Seite Kontaktflächen (27) hat, die mit den elektrischen Kontaktstellen (16) des Montageadapters (2) in Kontakt bringbar sind, wenn das Trägerelement (19) mit dem Montageadapter (2) zusammengesetzt wird, wobei aus der zu den Kontaktflächen (27) entgegengesetzten Seite der Trägerplatte (19) die elektronischen Bauteile (20, 21) angeordnet sind, die von den Kontaktstellen (16) über die Kontaktflächen (27) mit elektrischem Strom versorgt werden.

6. Endoskopkopf gemäß einem der vorstehenden Ansprüche, **dadurch gekennzeichnet, dass** des Halterungselement (22) für die Optik auf der die elektronischen Bauteile tragenden Seite des Trägerelements (19) an diesem befestigt ist und zumindest eine, die Optik aufnehmende Kammer (29) hat, die zu der der Schutzkappe zugewandten Seite des Halterungselements (22) hin offen ist und die sich beim Zusammenbau des Halterungselements (22) mit dem Trägerelement (19) über einem auf dem Trägerelement (19) befindlichen Kamerachip (21) ausrichtet.

7. Endoskopkopf gemäß einem der vorstehenden Ansprüche, **dadurch gekennzeichnet, dass** das Material des Halterungselements (23) weicher und/oder elastischer ist als das Material der Schutzkappe (22).

8. Endoskopkopf gemäß einem der vorstehenden Ansprüche, **gekennzeichnet durch** eine kippbare und/oder drehbare Spiegelungs- und/oder Prismenvorrichtung, die bezüglich der die Optik aufnehmenden Kammer so bewegbar ist, dass sie wahlweise Lichtstrahlen, die außerhalb des normalen Lichteinfallbereichs der in der Kammer befindlichen Optik liegen, auf die Optik umlenkt.

9. Endoskopkopf gemäß einem der Ansprüche 1 bis 8 **dadurch gekennzeichnet, dass** der Montageadapter (2) so ausgebildet, um zum einen eine mechanische Verbindung zwischen dem Endoskopkopf und einem Endoskopschaft zu bilden und zum anderen eine Verbindung zwischen in dem Endoskopschaft ausgebildeten Versorgungskanälen und Leitungen mit Funktionseinheiten des Endoskopkopfs zu schaffen.

10. Endoskopkopf nach Anspruch 9, **dadurch gekennzeichnet, dass** der Montageadapter aus einer äußeren, im Wesentlichen zylindrischen Hülse und einer coaxialen inneren, im wesentlichen zylindrischen Hülse besteht, die über profilierte Radialverstrebungen mit der äußeren Hülse vorzugsweise einstückig verbunden ist, wobei die Radialverstrebungen zumindest teilweise die Verbindung der Versorgungskanäle und Leitungen mit den Funktionseinheiten schaffen und/oder ermöglichen.

11. Endoskopkopf nach Anspruch 10, **gekennzeichnet durch** eine an einer Stirnseite des Montageadapters (2) ausgebildete montageplatte (10), auf der der Endoskopkopf montierbar ist, wobei die Montageplatte (10) mechanische und elektrische Anschlüsse für den Endoskopkopf ausweist.

12. Endoskopkopf gemäß Anspruch 11, **dadurch gekennzeichnet, dass** die elektrischen Anschlüsse als in der Montageplatte (10) ausgebildete flexible Kontaktarme (15) vorgesehen sind, die vorzugsweise über die Fläche der Montageplatte (10) hervorstehende Kontaktstellen (16) haben.

## Claims

1. Endoscope head which is equipped with a number of functional units, such as for example a lens system, illumination elements, rinsing nozzles and the like, the endoscope head comprising a number of modular functional carriers (19, 22, 23) for receiving and/or forming correspondingly assigned functional units (20, 21, 38) which form a plate-shaped carrier element (19) with electronic components (20, 21), namely an optical sensor chip (21)
a mounting element (22) for a lens system which has at least two lenses (38);
a protective cap (23) for covering the lens mounting element (22) and the carrier plate (19) and
an assembly adaptor (2) for mounting the endoscope head on an endoscope shaft, wherein the assembly adaptor (2) is configured in such a manner in order to create and/or to produce a connection between supply channels and lines configured in the endoscope shaft to the functional units of the endoscope head, **characterized in that** the protective cap (23) at least partially exists of a transparent material and that the electronic components (20, 21) further comprise illumination elements (20).

2. Endoscope head according to claim 1,
**characterized in that**
the functional carriers (19, 22, 23) are configured in such a manner that the functional elements (20, 21, 38) can be placed correctly and/or can be brought into functional capacity by assembling the functional carriers (19, 22, 23), preferably automatically.

3. Endoscope head according to claim 2,
**characterized in that**
the functional carriers (19, 22, 23) are configured respectively with connecting portions for a snap-in and/or clamping connection, which connecting portions can be brought in predetermined engagement with each other and can be deformed, preferably elastically.

4. Endoscope head according to claim 1,
**characterized in that**
the assembly adaptor (2) is configured with electrical contact points (16) to which electrical lines in the endoscope shaft can be connected.

5. Endoscope head according to claim 4,
**characterized in that**
the carrier element (19) has, on its side orientated towards the assembly adaptor (2), contact faces (27) which can be brought in contact with the electrical contact points (16) of the assembly adaptor (2) when the carrier element (19) is assembled with the assembly adaptor (2), the electronic components (20, 21) being disposed on the side of the carrier plate (19) which is opposite the contact faces (27), said electronic components being supplied with electrical current from the contact points (16) via the contact faces (27).

6. Endoscope head according to one of the preceding claims,
**characterized in that**
the mounting element (22) for the lens system on the side of the carrier element (19) which carries the electronic components is mounted on said carrier element and has at least one chamber (29) which receives the lens system, which chamber is open towards the side of the mounting element (22) orientated towards the protective cap and which, during assembly of the mounting element (22) with the carrier element (19), is orientated above a camera chip (21) which is situated on the carrier element (19).

7. Endoscope head according to one of the preceding claims,
**characterized in that**
the material of the mounting element (22) is softer and/or more elastic than the material of the protective cap (23).

8. Endoscope head according to one of the preceding claims,
**characterized by**
a tiltable and/or rotatable reflection and/or prism device which is moveable relative to the chamber which receives the lens system such that optionally it deflects onto the lens system light beams which are outwith the normal light incidence region of the lens system situated in the chamber.

9. Endoscope head according to one of the claims 1 to 8,
**characterised in that**
the assembly adaptor (2) is configured in such a manner in order, on the one hand, to form a mechanical connection between the endoscope head and an endoscope shaft and, on the other hand, to produce a connection between supply channels and lines which are configured in the endoscope shaft to functional units of the endoscope head.

10. Endoscope head according to claim 9,
**characterised in that**
the assembly adaptor comprises an outer, essentially cylindrical shell and a coaxial inner, essentially cylindrical shell which is connected preferably in one piece to the outer shell via profiled radial struts, the radial struts producing and/or enabling at least partially the connection of the supply channels and lines to the functional units.

11. Endoscope head according to claim 10,
**characterized by**
an assembly plate (10) which is configured on an end side of the assembly adaptor (2) and on which the endoscope head can be mounted, the assembly plate (10) having mechanical and electrical connections for the endoscope head.

12. Endoscope head according to claim 11,
**characterized in that**
the electrical connections are provided as flexible contact arms (15) which are configured in the assembly plate (10) and preferably have contact points (16) which project beyond the surface of the assembly plate (10).

## Revendications

1. Tête d'endoscope, équipée d'une pluralité d'unités fonctionnelles, telles que, par exemple, une optique, 1 des éléments d'éclairage, des buses de rinçage et analogues, la tête d'endoscope étant composée d'une pluralité de supports fonctionnels (19, 22, 23) modulaires, pour recevoir et/ou former des unités fonctionnelles (20, 21, 38), associées de manière correspondante, constituant un élément support (19) en forme de plaque, avec des composants électroniques (20, 21), précisément une puce électronique de capteur optique (21), un élément de fixation (22) pour une optique présentant au moins deux lentilles (38) ; un capuchon de protection (23) pour couvrir l'élément de maintien de lentilles (22) et la plaque support (19), et un adaptateur de montage (2) pour fixation de la tête d'endoscope sur une tige d'endoscope, dans laquelle l'adaptateur de montage (2) est réalisé de manière à créer et/ou établir une liaison entre des canaux d'alimentation et des conduites, formés dans la tige d'endoscope, avec les unités fonctionnelles de la tête d'endoscope, **caractérisée en ce que** le capuchon de protection (23) est composé au moins partiellement d'un matériau transparent à la lumière, et **en ce que** les composants électroniques (20, 21) comprennent en outre des corps éclairants (20).

2. Tête d'endoscope selon la revendication 1,
**caractérisée en ce que**
les supports fonctionnels (19, 22, 23) sont réalisés de manière que
les éléments fonctionnels (20, 21, 38) sont susceptibles d'être correctement placés et/ou sont susceptibles d'être placés en aptitude fonctionnelle, de préférence automatiquement, par assemblage des supports fonctionnels (19, 22, 23).

3. Tête d'endoscope selon la revendication 2,
**caractérisée en ce que**
les supports fonctionnels (19, 22, 23) sont réalisés avec chaque fois des tronçons de liaison, susceptibles d'être mis en prise de façon prédéterminée les uns avec les autres, de préférence déformables élastiquement, pour une liaison à encliquetage et/ou à serrage.

4. Tête d'endoscope selon la revendication 1,
**caractérisée en ce que**
l'adaptateur de montage (2) est réalisé avec des points de contact (16) électriques, et les lignes électriques sont susceptibles d'être raccordées dans la tige d'endoscope.

5. Tête d'endoscope selon la revendication 4,
**caractérisée en ce que**
l'élément support (19) comprend, sur sa face tournée vers l'adaptateur de montage (2), des faces de contact (27), susceptibles d'être mises en contact avec les points de contact (16) électriques de l'adaptateur de montage (2), lorsque l'élément support (19) est assemblé à l'adaptateur de montage (2), sachant que, sur le côté opposé aux faces de contact (27) de la plaque support (19), sont disposés les composants électroniques (20, 21) qui sont alimentés en courant électrique par les points de contact (16), par l'intermédiaire des faces de contact (27).

6. Tête d'endoscope selon l'une des revendications précédentes,
**caractérisée en ce que**
l'élément de fixation (22) pour l'optique, sur la face portant les composants électroniques de l'élément support (19), est fixé sur celui-ci, et comprend au moins une chambre (29) recevant l'optique, chambre ouverte vers le côté, tourné vers le capuchon de protection, de l'élément de fixation (22) et qui, lors de l'assemblage de l'élément de fixation (22), s'oriente avec l'élément support (19), par l'intermédiaire d'un chip de caméra (21) se trouvant sur l'élément support (19).

7. Tête d'endoscope selon l'une des revendications précédentes,
**caractérisée en ce que**
le matériau de l'élément de fixation (23) est plus souple et/ou plus élastique que le matériau du capuchon de protection (22).

8. Tête d'endoscope selon l'une des revendications précédentes,
**caractérisée par**
un dispositif de réflexion et/ou à prisme, susceptible d'être incliné et/ou susceptible d'être tourné, déplaçable par rapport à la chambre recevant l'optique, de manière qu'il dévie sur l'optique, au choix, des rayons lumineux situés hors de la plage d'incidence lumineuse normale de l'optique se trouvant dans la chambre.

9. Tête d'endoscope selon l'une des revendications 1 à 8,
**caractérisée en ce que**
l'adaptateur de montage (2) est réalisé pour, d'une part, former une liaison mécanique entre la tête d'endoscope et une tige d'endoscope et pour, d'autre part, créer une liaison entre des canaux d'alimentation et des conduites, réalisés dans la tige d'endoscope, avec des unités fonctionnelles de la tête d'endoscope.

10. Tête d'endoscope selon la revendication 9,
**caractérisée en ce que**
l'adaptateur de montage est formé d'une douille extérieure sensiblement cylindrique et d'une douille intérieure coaxiale sensiblement cylindrique, reliées, de préférence, d'une seule pièce à la douille extérieure, par l'intermédiaire d'entretoisements radiaux profilés, les entretoisements radiaux créant et/ou permettant, au moins partiellement, la liaison des canaux d'alimentation et des conduites avec les unités fonctionnelles.

11. Tête d'endoscope selon la revendication 10,
**caractérisée par**
une plaque de montage (10) réalisée sur une face frontale de l'adaptateur de montage (2), plaque de montage sur laquelle la tête d'endoscope peut être montée, la plaque de montage (10) présentant des raccordements mécaniques et électriques pour la tête d'endoscope.

12. Tête d'endoscope selon la revendication 11,
**caractérisée en ce que**
les raccordements électriques sont prévus sous la forme de bras de contact (15) flexibles, réalisés dans la plaque de montage (10), ayant, de préférence, des points de contact (16) en saillie sur la surface de la plaque de montage (10).
